Europäisches Patentamt

European Patent Office

Office européen des brevets

(19)

(11) Veröffentlichungsnummer : **0 463 011 B1**

(12) **EUROPÄISCHE PATENTSCHRIFT**

(45) Veröffentlichungstag der Patentschrift :
20.01.93 Patentblatt 93/03

(51) Int. Cl.⁵ : **A61F 2/30**

(21) Anmeldenummer : 90904332.5

(22) Anmeldetag : 07.03.90

(86) Internationale Anmeldenummer :
PCT/EP90/00368

(87) Internationale Veröffentlichungsnummer :
WO 90/11062 04.10.90 Gazette 90/23

(54) **KÜNSTLICHES GELENK.**

(30) Priorität : 18.03.89 DE 3908958

(43) Veröffentlichungstag der Anmeldung :
02.01.92 Patentblatt 92/01

(45) Bekanntmachung des Hinweises auf die
Patenterteilung :
20.01.93 Patentblatt 93/03

(84) Benannte Vertragsstaaten :
AT BE CH DE DK ES FR GB IT LI LU NL SE

(56) Entgegenhaltungen :
FR-A- 2 085 013
US-A- 29 757
US-A- 3 916 451
US-A- 4 003 095

(73) Patentinhaber : **Kubein-Meesenburg, Dietmar**
**Burgweg 1a**
**W-3350 Kreiensen (DE)**
Patentinhaber : **NÄGERL, Hans**
**Lange Hecke 41**
**W-3407 Gleichen/OT Klein Lengden (DE)**

(72) Erfinder : **Kubein-Meesenburg, Dietmar**
**Burgweg 1a**
**W-3350 Kreiensen (DE)**
Erfinder : **NÄGERL, Hans**
**Lange Hecke 41**
**W-3407 Gleichen/OT Klein Lengden (DE)**

(74) Vertreter : **Zapf, Christoph et al**
**Patentanwälte Dr. Solf und Zapf**
**Schlossbleiche 20**
**W-5600 Wuppertal 1 (DE)**

**Beschreibung**

Die vorliegende Erfindung betrifft ein künstliches Gelenk zum Ersatz insbesondere von menschlichen Gelenken, bestehend aus mindestens zwei Gelenkteilen mit zueinander sich bewegenden sphärischen Funktionsflächen, wobei die Krümmungsverhältnisse der eine kreisförmige Schnittkontur aufweisenden Funktionsflächen zueinander konvex-konkav derart ausgebildet sind, daß ihre Rotationszentren $M_1$ und $M_2$ innerhalb des Gelenkteiles mit der konvexen Funktionsfläche liegen, und zwischen den beiden Funktionsflächen ist ein Druckverteilungskörper angeordnet, dessen an den Funktionsflächen anliegende Gleitflächen eine den Funktionsflächen entsprechend angepaßte Krümmung aufweisen, wobei die Gelenkgeometrie durch eine Gelenkkette mit zwei Gelenkachsen bestimmt ist, die durch die Rotationszentren $M_1$ und $M_2$ der Funktionsflächen mit den Radien $R_1$ und $R_2$ verlaufen, wobei $R_1$ der Radius der kreisförmigen Schnittkontur der Funktionsfläche mit dem Mittelpunkt $M_1$ und $R_2$ der Radius der kreisförmigen Schnittkontur der Funktionsfläche mit dem Mittelpunkt $M_2$ ist.

In jedem menschlichem Gelenk gleiten und rollen glatte und geschmierte Funktionsflächen aneinander ab, deren Bewegung wegen der Flüssigkeitsschmierung, nur durch geringe, geschwindigkeitsabhängige Reibungskräfte beeinflußt wird. Gibt es eine besonders ausgeprägte Funktionsrichtung des Gelenks, so vollführt der vom Gelenk geführte Körperteil am häufigsten eine ebene Bewegung aus, relativ zu dem mit ihm verbundenen, als ruhend betrachteten Teil. Um diese Bewegung herum bleibt nach beiden Seiten der allgemeine Bewegungsraum auf einen relativ kleinen Winkelbereich beschränkt (beispielsweise Kiefergelenk, Kniegelenk, scharnierartige Gelenke). Es hat sich herausgestellt, daß die bekannten künstlichen Gelenke nicht ausreichend sind, um die Funktionen der natürlichen Gelenke nachzubilden, so daß sie einem frühen Verschleiß unterliegen und zu Behinderungen beim Menschen führen.

Aus der US-A-3916451 ist ein künstliches Gelenk zum Ersatz von menschlichen Gelenken bekannt, das aus mindestens zwei Gelenkteilen mit zueinander sich bewegenden, sphärischen Funktionsflächen besteht. Bei diesem Gelenk sind die Krümmungsverhältnisse der eine kreisförmige Schnittkontur aufweisenden Funktionsflächen zueinander konvex-konkav und die Gelenkgeometrie ist durch eine Gelenkkette mit zwei Gelenkachsen bestimmt, die durch die beiden Rotationszentren der Funktionsflächen mit den entsprechenden Radien verlaufen. Bei dem aus dieser Druckschrift bekannten Gelenksystem handelt es sich jedoch um ein druckinstabiles Gelenksystem, das lediglich zugstabil ist. Derartige lediglich zugstabile Gelenksysteme sind aber zum Einsatz als künstliche Gelenke im menschlichen Körper nicht brauchbar, da dort druckstabile Systeme erforderlich sind.

Der Erfindung liegt die Aufgabe zugrunde, künstliche Gelenke zu schaffen, die einen Aufbau aufweisen, der eine den natürlichen Gelenken weitestgehend entsprechende Funktion sicherstellt und somit eine beschwerde- und behinderungsfreie Funktion während eines langen Zeitraumes im Menschen bewirken.

Erfindungsgemäß wird dies durch ein künstliches Gelenk erreicht, bei dem der Druckverteilungskörper eine Dicke D auf der Verbindungslinie der Rotationszentren $M_1$ und $M_2$ aufweist, und zwar derart, daß die Gelenkachsenbahn der Rotationszentren $M_1$, $M_2$ einen Radius $R = R_2 - R_1 - D$ besitzt.

Die Erfindung basiert auf der Erkenntnis, daß das Abgleiten zweier sphärischer Funktionsflächen aneinander für die ebene Bewegung auf das Abgleiten zweier Kurven aneinander reduziert werden kann. Diese beiden Kurven werden erfindungsgemäß durch die beiden Schnittkonturen durch die Gelenkoberflächen gebildet.

Weitere vorteilhafte Ausführungen der Erfindung sind in den Unteransprüchen enthalten.

Anhand der in den beiliegenden Zeichnungen dargestellten Ausführungsbeispiele wird die Erfindung nunmehr näher erläutert. Es zeigen:

Fig. 1 eine Prinzipdarstellung der Funktionsflächen eines Teil-Gelenks mit Doppelkonvexität eines erfindungsgemäßen Gelenksystems,

Fig. 2 eine Prinzipansicht der Funktionsflächen eines Teil-Gelenks mit Doppelkonkavität eines erfindungsgemäßen Gelenksystems,

Fig. 3 eine Prinzipansicht der Funktionsflächen eines erfindungsgemäßen Gelenks bei Konvexität-Konkavität,

Fig. 4 eine Prinzipansicht der Funktionsflächen eines Teil-Gelenks eines erfindungsgemäßen Gelenksystems,

Fig. 5 eine Prinzipdarstellung eines erfindungsgemäßen Gelenks als Hüftgelenk eines Menschen,

Fig. 6 eine Prinzipdarstellung eines erfindungsgemäßen Gelenksystems als Kniegelenk.

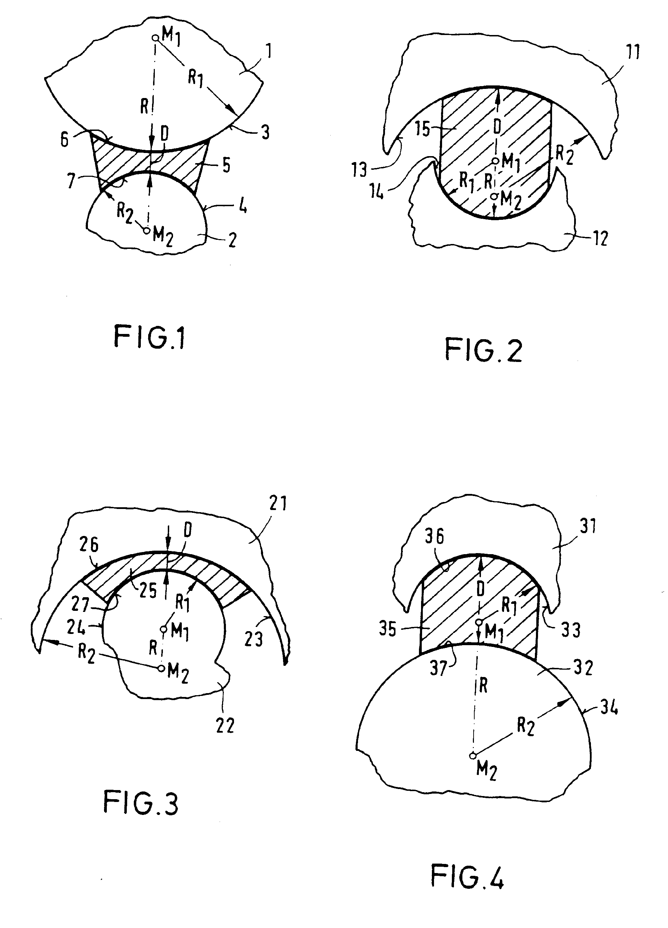

Wie sich aus Fig. 1 ergibt, besteht ein Jeil-Gelenk aus einem Gelenkteil 1 und einem Gelenkteil 2. Im dargestellten Ausführungsbeispiel besitzen die Gelenkteile 1 und 2 jeweils konvexe Funktionsflächen 3 und 4. Diese Funktionsflächen 3 und 4 haben eine kreisförmige Schnittkontur und die Funktionsfläche 1 besitzt ein Rotationszentrum $M_1$ und die Funktionsfläche 2 besitzt ein Rotationszentrum $M_2$. Die kreisförmige Schnittkontur der Funktionsfläche 1 hat den Radius $R_1$ und die kreisförmige Schnittkontur der Funktionsfläche 4 hat den Radius $R_2$. Die Gelenkachsenbahn des Gelenkes besitzt einen Radius R, der sich aus der folgenden Beziehung ergibt.

$$R = R_1 + R_2 + D$$

Hierbei stellt D die Dicke eines Druckverteilungskörpers 5 dar, und zwar auf der Verbindungslinie der beiden Rotationszentren $M_1$ und $M_2$. Der Druckverteilungskörper 5 weist Gleitflächen 6, 7 auf, die jeweils eine den Funktionsflächen 3 und 4 entsprechend angepaßte Krümmung besitzen. Die Dicke D des Druckverteilungskörpers 5 ist abhängig von der Belastung im Gelenk. Der Radius R der Gelenkachsenbahn ist vorgegeben, d.h., er wird aus dem natürlichen Gelenk, das durch das künstliche Gelenk ersetzt werden soll, bestimmt. Die Radien $R_1$ oder $R_2$ sind alternativ am natürlichen Gelenk nachmeßbar, so daß sich somit, aufgrund der einzelnen Parameter, ein am natürlichen Gelenk orientiertes künstliches Gelenk aufbauen läßt. Grundsätzlich ist der Radius R die das künstliche und natürliche Gelenk bestimmende Konstante. Das künstliche Gelenk kann abweichend vom natürlichen mit anderen Radii $R_1$, $R_2$ und D aufgebaut werden, wobei die Radiensumme R dieselbe bleibt, um die verwendeten künstlichen Materialien der Belastungsoptimierung anzupassen.

Das Gelenk ist demnach wie eine Gelenkkette mit zwei Gelenkachsen aufgebaut, d.h. es handelt sich um eine sogenannte "dimere" Gelenkkette.

Die technische Ausführung einer derartigen "dimeren" Gelenkkette entspricht einer Ausführung aus zwei Rundlingen, die über ein Gestänge mit dem Abstand R voneinander gehalten werden.

An den beweglichen Enden der Gelenkkette sind dann jeweils die beiden gelenkig verbundenen Körper befestigt. Wesentlich ist bei der Ausführung des Gelenkes gemäß Fig. 1, daß die beiden Rotationszentren $M_1$ und $M_2$ jeweils in ihrem zugehörigen Gelenkteil 1 bzw. Gelenkteil 2 liegen. Die Aufgabe des Druckverteilungskörpers 5 liegt darin, die im Gelenk auftretenden Kräfte über die Funktionsflächen des Gelenkes zu verteilen, so daß die Kontaktflächen vergrößert werden, um punktförmige Belastungen zu vermeiden.

In Fig. 2 ist ein Teil-Gelenk dargestellt, daß aus den Gelenkteilen 11 und 12 besteht. Diese Gelenkteile 11 und 12 besitzen konkave Funktionsflächen 13, 14 mit kreisförmiger Schnittkontur und zwischen den Gelenkteilen 11, 12 ist ein Druckverteilungskörper 15 angeordnet. Der Radius R der Gelenkachsenbahn dieses Gelenks ergibt sich aus der Beziehung

$$R = R_2 + R_1 - D,$$

wobei D wiederum die Dicke des Druckverteilungskörpers 15 auf der Verbindungslinie der Rotationszentren $M_1$ und $M_2$ liegt, wobei die Rotationszentren $M_1$ und $M_2$ innerhalb des Druckverteilungskörpers liegen. $R_1$ ist der Radius der kreisförmigen Schnittkontur um das Rotationszentrum $M_1$ und $R_2$ der Radius der kreisförmigen Schnittkontur um das Rotationszentrum $M_2$.

In Fig. 3 ist eine Ausbildung eines erfindungsgemäßen Gelenks im Prinzip dargestellt, wobei die beiden Gelenkkörper 21, 22 unterschiedliche sphärische Krümmungen aufweisen. Der Gelenkkörper 21 weist eine konkave Funktionsfläche 23 auf und der Gelenkkörper 22 eine konvexe Funktionsfläche 24.

Zwischen den Gelenkteilen 21, 22 ist wiederum ein Druckverteilungskörper 25 angeordnet. Dieser Druckverteilungskörper besitzt die Gleitflächen 26, 27. Das Gelenkteil 22 besitzt das Rotationszentrum $M_1$ und die kreisförmige Schnittkontur der Funktionsfläche 24 des Gelenkteils 22 besitzt den Radius $R_1$. Das Gelenkteil 21 besitzt das Rotationszentrum $M_2$ und seine konkave Funktionsfläche 23 weist in ihrer kreisförmigen Schnittkontur den Radius $R_2$ auf. Hierbei sind die Rotationszentren innerhalb des Gelenkteils mit der konvexen Funktionsfläche 24 angeordnet. Der Radius R der Gelenkachsenbahn ergibt sich aus der Beziehung

$$R = R_2 - R_1 - D$$

Hierbei ist D wiederum die Dicke des Druckverteilungskörpers 25 auf der Verbindungslinie der Rotationszentren $M_1$ und $M_2$.

In Figur 4 is eine alternative Ausführungsform eines Teil-Gelenks mit den Gelenkteilen 31, 32 dargestellt. Auch hier weist das eine Gelenkteil, nämlich das Gelenkteil 31, eine konkave Funktionsfläche 33 auf und das Gelenkteil 32, eine konvexe Funktionsfläche 34. Im Gegensatz zu der Ausführungsform gemäß Fig. 3 besitzt hier die kreisförmige Schnittkontur der konvexen Funktionsfläche einen größeren Radius, nämlich den Radius $R_2$ mit dem Rotationszentrum $M_2$ und das Gelenkteil 31 die konkave Funktionsfläche 33 mit einem gegenüber dem der Funktionsfläche 23 in Fig. 3 kleineren Radius $R_1$ mit dem Rotationszentrum $M_1$. Die Rotationszentren sind derart angeordnet, daß das Rotationszentrum des Gelenkteils 35 in ihm selber liegt und das Rotationszentrum des Gelenkteils 31 innerhalb des Druckverteilungskörpers 35, der zwischen den beiden Gelenkteilen 31 und 32 angeordnet ist. Der Druckverteilungskörper 35 besitzt die an den Funktionsflächen 33 bzw. 34 anliegenden Gleitflächen 36, 37. Der Radius R der Gelenkachsenbahn ergibt sich aus der Beziehung

$$R = R_2 - R_1 + D$$

Die Gelenkachsenbahnen der einzelnen in den Figuren 1 bis 4 dargestellten Gelenke, ist dabei jeweils diejenige Bahn, und zwar eine Kreisbahn, mit der sich die Mittelpunkte $M_1$ bzw. $M_2$ in Abhängigkeit des jeweiligen Bezugssystems um den anderen Rotationsmittelpunkt bewegen. Diese Bewegungen der Mittelpunkte $M_1$ und $M_2$ sind unabhängig von zusätzlichen Rotationen der Funktionsflächen um Ihre eigenen Mittelpunkte.

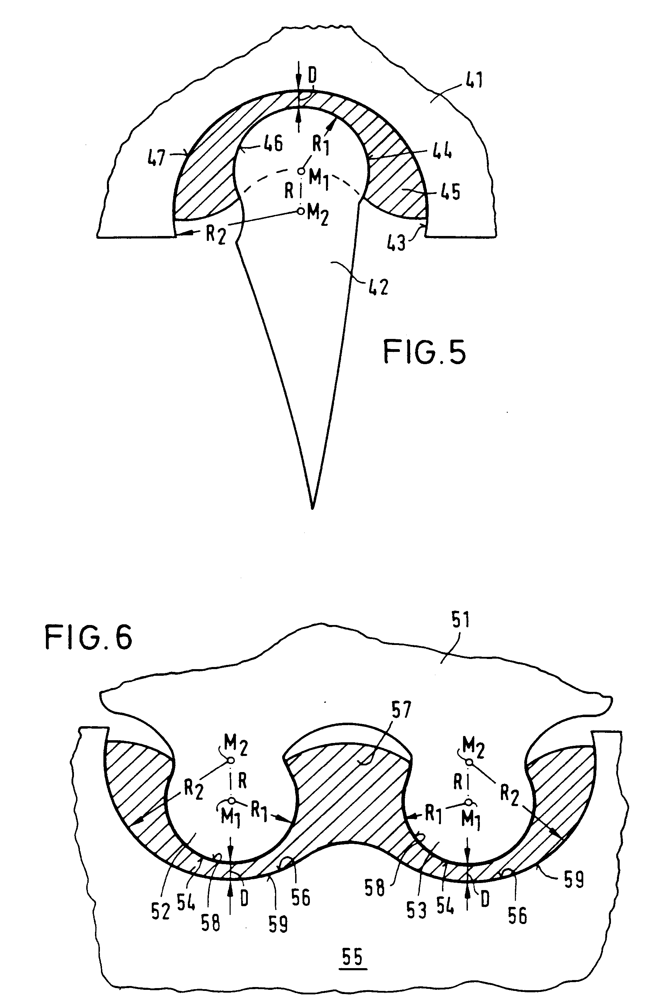

In Fig. 5 ist die Ausführungsform eines erfindungsgemäßen Gelenks als menschliches Hüftgelenk dargestellt. Hierbei handelt es sich um eine Anwendung der Gelenkausbildung gemäß Fig. 3. D.h. es liegt der Fall Konvexität-Konkavität vor. Hierbei weist das Hüftbein 41 als konkave Funktionsfläche die Pfanne 43 auf. Der Rotationsmittelpunkt des Hüftbeins bzw. der Pfanne ist $M_2$. Und der Radius der kreisförmigen Funktionsfläche der Pfanne ist $R_2$. Das andere Gelenkteil wird von dem Femur 42 gebildet, der eine konvexe Funktionsfläche 44 besitzt, mit dem Rotatiönsmittelpunkt $M_1$ und dem Radius $R_1$ als Krümmungsradius. Zwischen den beiden Gelenkteilen 41, 42 ist der Druckverteilungskörper 45 angeordnet, dessen an den Funktionsflächen 43, 44 anliegenden Gleitflächen 45, 46 den Funktionsflächen entsprechende und angepaßte Krümmungen aufweisen. Vorteilhaft ist die Ausbildung des Druckverteilungskörpers 45 derart, daß er über die Mitte des halbkugelförmigen Teils des Femurs 42 gezogen ist, so daß das dargestellte, erfindungsgemäße Gelenk auch etwas Zug aushalten kann und z.B. nicht aufgrund der Schwerkraft des Beines bzw. des Unterschenkels auseinanderfallen kann. Was auch in bezug auf die äußere Gleitfläche 47 des Druckverteilungskörpers 45 gilt, die ebenfalls über die Hälfte der halbkugelförmigen Funktionsfläche 43 hinaus gezogen ist.

Erfindungsgemäß ist dabei vorgesehen, daß dieses Einklemmen des Gelenks mittels des Druckverteilungskörpers in die von der Funktionsfläche 43 gebildete Hohlkugel nicht überall rundherum geschehen muß.

In Fig. 6 ist die Außenbildung eines Gelenksystems dargestellt, wie es für die Nachbildung des menschlichen Kniegelenks erforderlich ist. Dieses Gelenksystem besteht aus einer parallelschaltung zweier erfindungsgemäßer Gelenke entsprechend der Gelenkausbildung von Fig. 3. Hierbei sind dem Femur 51 zwei Gelenkteile 52, 53 ausgebildet, die parallel zueinander angeordnet sind, die jeweils eine konvexe Funktionsfläche 54 aufweisen. Die konvexen Funktionsflächen 54 besitzen einen Rotationsmittelpunkt $M_1$ und einen Radius $R_1$ ihrer kreisförmigen Schnittkontur. In der Tibea 55 sind zwei Funktionsflächen 56 parallel zueinander angeordnet, die konkav ausgebildet sind, und deren Rotationsmittelpunkt $M_2$ innerhalb des Femur 51 liegt. Diese Funktionsflächen 56 besitzen jeweils eine kreisförmige Schnittkontur mit dem Radius $R_2$. Zwischen den Funktionsflächen bzw. den Gelenkteilen 51, 55 ist ein Druckverteilungskörper 57 angeordnet. Dieser Druckverteilungskörper ist derart ausgestaltet, daß seine Gleitflächen 58, 59 jeweils über die Hälfte der halbkugelförmigen Funktionsflächen der Gelenkteile 51, 55 hinaus verlängert sind, so daß die Gelenkteile 51, 55 klemmend gehalten sind.

Weiterhin ist es erfindungsgemäß möglich, aus den Teilgelenken der Fig. 1, 2 und 4 und dem erfindungsgemäßen Gelenk gemäß Fig. 3 durch Parallel- oder Hintereinanderschaltung ein Gelenksystem zu bilden.

**Patentansprüche**

1. Künstliches Gelenk zum Ersatz insbesondere von menschlichen Gelenken, bestehend aus mindestens zwei Gelenkteilen (21, 22; 41, 43; 51, 55) mit zueinander sich bewegenden sphärischen Funktionsflächen (23, 24; 43, 44; 54, 56), wobei die Krümmungsverhältnisse der eine kreisförmige Schnittkontur aufweisenden Funktionsflächen (23, 24; 43, 44; 54, 56) zueinander konvexkonkav derart ausgebildet sind, daß ihre Rotationszentren $M_1$ und $M_2$ innerhalb des Gelenkteiles (21; 42; 52, 53) mit der konvexen Funktionsfläche (24; 44; 54) liegen und zwischen den beiden Funktionsflächen ist ein Druckverteilungskörper (25; 45; 57) angeordnet, dessen an den Funktionsflächen anliegende Gleitflächen (26, 27; 45, 46; 58, 59) eine den Funktionsflächen (23, 24; 43, 44; 54, 56) entsprechend angepaßte Krümmung aufweisen, wobei die Gelenkgeometrie durch eine Gelenkkette mit zwei Gelenkachsen bestimmt ist, die durch die Rotationszentren $M_1$ und $M_2$ der Funktionsflächen (23, 24; 43, 44; 54, 56) mit den Radien $R_1$ und $R_2$ verlaufen, wobei $R_1$ der Radius der kreisförmigen Schnittkontur der Funktionsfläche (24; 44; 54) mit dem Mittelpunkt $M_1$ und $R_2$ der Radius der kreisförmigen Schnittkontur der Funktionsfläche (23; 43; 56) mit dem Mittelpunkt $M_2$ ist, **dadurch gekennzeichnet,** daß der Druckverteilungskörper (25; 45; 57) eine Dicke D auf der Verbindungslinie der Rotationszentren $M_1$ und $M_2$ aufweist, und zwar derart, daß die Gelenkachsenbahn der Rotationszentren ($M_1$, $M_2$) einen Radius $R = R_2 - R_1 - D$ besitzt.

2. Gelenksystem aus mindestens zwei künstlichen Gelenken, jedes bestehend aus mindestens zwei Gelenkteilen mit zueinander sich bewegenden sphärischen Funktionsflächen, **gekennzeichnet durch** eine Parallelschaltung von mindestens zwei Gelenken nach Anspruch 1.

3. Gelenksystem gemäß dem Oberbegriff des Anspruchs 2, **gekennzeichnet durch** eine Serienschaltung mindestens zweier Gelenke nach Anspruch 1.

4. Gelenksystem bestehend aus mindestens zwei künstlichen Gelenken, jedes bestehend aus mindestens zwei Gelenkteilen mit zueinander sich bewegenden sphärischen Funktionsflächen, **gekennzeichnet durch** eine Parallel- oder Serienschaltung eines Gelenks nach Anspruch 1 mit einem Gelenk, dessen Funktionsflächen (3, 4) der Gelenkteile (1, 2) konvex ausgebildet sind und ihre Rotationszentren $M_1$ und $M_2$ in dem zugehörigen Gelenkteil (1, 2) liegen und ihre Gelenkachsenbahn einen Radius $R = R_1 + R_2 + D$ besitzt.

5. Gelenksystem bestehend aus mindestens zwei künstlichen Gelenken, jedes bestehend aus mindestens zwei Gelenkteilen mit zueinander sich bewegenden sphärischen Funktionsflächen, **gekennzeichnet durch** eine Parallel- oder Serienschaltung eines Gelenks nach Anspruch 1 mit einem Gelenk, bei dem die beiden Funktionsflächen (13, 14) konkav ausgebildet sind und ihre Rotationszentren $M_1$ und $M_2$ im Druckverteilungskörper (15) liegen und ihre Gelenkachsenbahn den Radius $R = R_2 + R_1 - D$ besitzt.

6. Gelenksystem bestehend aus mindestens zwei künstlichen Gelenken, jedes bestehend aus mindestens zwei Gelenkteilen mit zueinander sich bewegenden sphärischen Funktionsflächen, **gekennzeichnet durch** eine Parallel- oder Serienschaltung eines Gelenks nach Anspruch 1 mit einem Gelenk, bei dem eine Funktionsfläche (34) eines Gelenkteils (32) konvex und die andere Funktionsfläche (33) des Gelenkteils (31) konkav ausgebildet ist und das Rotationszentrum $M_1$ des Gelenkteils (34) mit der konvexen Funktionsfläche in diesem und das Rotationszentrum $M_1$ des Gelenkteils (31) mit der konkaven Funktionsfläche im Druckverteilungskörper (35) liegt und ihre Gelenkachsenbahn einen Radius $R = R_2 - R_1 + D$ besitzt.

## Claims

1. Artificial joint especially for replacing human joints, consisting of at least two joint parts (21,22; 41,43; 51,55) which move with respect to one another, wherein the radii of curvature of the operational faces having a circular intersection contour (23,24; 43,44; 54,56) are formed convex/concave one to the other in such a manner that their centres of rotation $M_1$ and $M_2$ are located inside the joint part (21; 42; 52; 53) with their convex operational faces (24; 44; 54) and a pressure distribution member (25; 45; 57) is arranged between the two operational faces and the sliding faces (26,27; 46,47; 58,59) of which, adjacent to the operational faces, have a curvature matched according to the operational faces (23,24; 43; 44; 54; 56), wherein the joint geometry is determined by a joint chain having two joint axes which pass through the rotation centres $M_1$ arid $M_2$ of the operational face (23, 24; 43,44; 54, 56) having the radii $R_1$ and $R_2$, wherein $R_1$ is the radius of the circular intersection contour of the operational faces (24; 44; 54) having the mid point $M_1$ and $R_2$ is the radius of the circular intersection contour of the operational face (23; 43; 56) having the mid point $M_2$, characterised in that the pressure distribution member (25; 45; 57) has a thickness D on the connection line of the rotation centres $M_1$ and $M_2$, and in such a manner that the joint axis path of the rotation centres ($M_1$, $M_2$) has a radius $R = R_2 - R_1 - D$.

2. Joint system consisting of at least two artificial joints, each consisting of at least two joint parts having spherical operational faces moving with respect to one another, characterised by a parallel connection of at least two joints according to Claim 1.

3. Joint system according to the preamble of Claim 2, characterised by a series connection of at least two joints according to Claim 1.

4. Joint system consisting of at least two artificial joints, each consisting of at least two joints having spherical operational faces moving with respect to one another, characterised by a parallel or series connection of a joint according to Claim 1 with a joint whose operational faces (3,4) of the joint parts (1,2) are formed convex and their rotation centres $M_1$ and $M_2$ lie in the mating joint part (1,2) and their joint axis part has a radius $R - R_1 + R_2 + D$.

5. Joint system consisting of at least two artificial joints, each consisting of at least two joint parts having spherical operational faces moving with respect to one another, characterised by a parallel or series connection of a joint according to Claim 1 with a joint in which the two function faces (13,14) are formed concave and their rotation centres $M_1$ and $M_2$ lie in the pressure distribution member (15) and their joint axis path has the radius $R = R_2 + R_1 - D$.

6. Joint system consisting of at least two artificial joints, each consisting of at least two joint parts having spherical operational faces moving with respect to one another, characterised by a parallel or series connection of a joint according to Claim 1 with a joint in which one operational face (34) of a joint part (32) is formed convex and the other operational face (33) of a joint part (31) is formed concave and the rotation centre $M_2$ of the joint part (34) having the convex operational face lies therein and the rotation centre $M_1$ of the joint part (31) having the concave operational face lies in the pressure distribution member (35) and their joint axis path has a radius $R = R_2 - R_1 + D$.

## Revendications

1. Articulation artificielle destinée à remplacer en particulier des articulations humaines, constituée d'au moins deux parties d'articulation (21, 22 ; 41, 43 ; 51, 55) comprenant des surfaces actives sphériques (23, 24 ; 43, 44 ; 54, 56) se déplaçant l'une par rapport à l'autre, les rapports de courbure entre les surfaces actives (23, 24 ; 43, 44 ; 54, 56) présentant un contour de section circulaire étant du type « convexe-concave », de façon que leurs centres de rotation $M_1$ et $M_2$ se trouvent à l'intérieur de la partie d'articulation (21 ; 42 ; 52, 53) à surface active convexe (24 ; 44 ; 54), et entre les deux surfaces actives étant disposé un corps (25 ; 45 ; 57) de répartition des pressions dont les surfaces de glissement (26, 27 ; 46, 47 ; 58, 59) appliquées contre les surfaces actives présentent une courbure adaptée aux surfaces actives (23, 24 ; 43, 44 ; 54, 56), la géométrie d'articulation étant définie par une chaîne articulée à deux axes d'articulation qui passent par les centres de rotation $M_1$ et $M_2$ des surfaces actives (23, 24 ; 43, 44 ; 54, 56) avec les rayons $R_1$ et $R_2$, $R_1$ étant le rayon du contour de section circulaire de la surface active (24 ; 44 ; 54) dont le centre est $M_1$, et $R_2$ étant le rayon du contour de section circulaire de la surface active (23 ; 43 ; 56) dont le centre est $M_2$, caractérisée en ce que le corps (25 ; 45 ; 57) de répartition des pressions présente une épaisseur D sur la ligne de jonction des centres de rotation $M_1$ et $M_2$, de façon que la trajectoire des axes d'articulation des centres de rotation ($M_1$, $M_2$) possède un rayon $R = R_2 - R_1 - D$.

2. Système d'articulation comprenant au moins deux articulations artificielles, constituées chacune d'au moins deux parties d'articulation comprenant des surfaces actives sphériques se déplaçant l'une par rapport à l'autre, caractérisé par un montage en parallèle d'au moins deux articulations conformes à la revendication 1.

3. Système d'articulation conforme au préambule de la revendication 2, caractérisé par un montage en série d'au moins deux articulations conformes à la revendication 1.

4. Système d'articulation comprenant au moins deux articulations artificielles, constituées chacune d'au moins deux parties d'articulation comprenant des surfaces actives sphériques se déplaçant l'une par rapport à l'autre, caractérisé par un montage en parallèle ou en série d'une articulation conforme à la revendication 1 avec une articulation dont les surfaces actives (3, 4) des parties d'articulation (1, 2) sont de forme convexe, leurs centres de rotation $M_1$ et $M_2$ se trouvant dans la partie d'articulation correspondante (1, 2), et la trajectoire de leur axes d'articulation possédant un rayon $R = R_1 + R_2 + D$.

5. Système d'articulation comprenant au moins deux articulations artificielles, constituées chacune d'au moins deux parties d'articulation comprenant des surfaces actives sphériques se déplaçant l'une par rapport à l'autre, caractérisé par un montage en parallèle ou en série d'une articulation conforme à la revendication 1 avec une articulation dans laquelle les deux surfaces actives (13, 14) sont de forme concave, leurs centres de rotation $M_1$ et $M_2$ se trouvant dans le corps (15) de répartition des pressions, et la trajectoire de leurs axes d'articulation possédant le rayon $R = R_2 + R_1 - D$.

6. Système d'articulation comprenant au moins deux articulations artificielles, constituées chacune d'au moins deux parties d'articulation comprenant des surfaces actives sphériques se déplaçant l'une par rapport à l'autre, caractérisé par un montage en parallèle ou en série d'une articulation conforme à la revendication 1 avec une articulation dans laquelle une surface active (34) d'une partie d'articulation (32) est de forme convexe et l'autre surface active (33) de la partie d'articulation (31) est de forme concave, le centre de rotation $M_2$ de la partie d'articulation (34) à surface active convexe se trouvant dans ladite partie d'articulation (34), le centre de rotation $M_1$ de la partie d'articulation (31) à surface active concave se trouvant dans le corps (35) de répartition des pressions, et la trajectoire de leurs axes d'articulation possédant un rayon $R = R_2 - R_1 + D$.

FIG.1

FIG.2

FIG.3

FIG.4

FIG.5

FIG.6